(19) Europäisches Patentamt
European Patent Office
Office européen des brevets

(11) Veröffentlichungsnummer: **0 507 177 B1**

# (12) EUROPÄISCHE PATENTSCHRIFT

(45) Veröffentlichungstag der Patentschrift: **13.12.95**

(51) Int. Cl.$^{6}$: **A61K 6/093**, C09J 183/06

(21) Anmeldenummer: **92104977.1**

(22) Anmeldetag: **23.03.92**

## (54) Provisorische Befestigungsmaterialien

(30) Priorität: **04.04.91 DE 4110796**

(43) Veröffentlichungstag der Anmeldung:
**07.10.92 Patentblatt 92/41**

(45) Bekanntmachung des Hinweises auf die Patenterteilung:
**13.12.95 Patentblatt 95/50**

(84) Benannte Vertragsstaaten:
**AT BE CH DE DK ES FR GB GR IT LI LU NL PT SE**

(56) Entgegenhaltungen:
**EP-A- 0 058 514**
**GB-A- 122 568**
**US-A- 3 574 943**

(73) Patentinhaber: **BAYER AG**
**D-51368 Leverkusen (DE)**

(72) Erfinder: **Schwabe, Peter, Dr.**
**Dudweiler Strasse 17**
**W-5090 Leverkusen (DE)**
Erfinder: **Schlak, Ottfried, Dr.**
**Massliebchenweg 17**
**W-5000 Köln 80 (DE)**
Erfinder: **Winkel, Jens, Dr.**
**Letterhausstrasse 1**
**W-5000 Köln 71 (DE)**
Erfinder: **Die anderen Erfinder haben auf ihre Nennung verzichtet**

EP 0 507 177 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Provisorische Befestigungsmaterialien werden in der Zahnarztpraxis häufig benutzt, z.B. zum Befestigen von provisorischen Kronen und Brücken, um die Patienten in der Zeit zu versorgen, die der Zahntechniker benötigt, um den endgültigen Zahnersatz herzustellen. Häufig werden aber auch definitive Kronen und Brücken eine gewisse Zeit probegetragen, um die Funktionsfähigkeit vor der endgültigen Eingliederung zu überprüfen. Auch in diesen Fällen werden die Kronen und Brücken mit provisorischen Zementen befestigt. Um als provisorischer Befestigungszement geeignet zu sein, muß das Material den Kaudruckbelastungen während der gesamten Tragezeit widerstehen, anderseits aber leicht entfernbar sein. Es muß speichelunlöslich sein, die Dentinwunde dicht verschließen, er darf keine Reizwirkung besitzen und es sollte vom Zahnarzt problemlos zu verarbeiten sein.

Bisher werden für diese Indikation hauptsächlich Zinkoxid-Eugenolzemente verwendet. Bei Bedarf vermischt der Zahnarzt eine zinkoxidhaltige Paste mit einer Eugenolpaste. Die Mischung härtet in ca. 2 - 3 min. aus.

Wenn der Zahnarzt das Material nicht korrekt dosiert oder die beiden Pasten unvollständig homogenisiert, so härtet der Zement nicht vollständig aus und hat keine ausreichende Festigkeit. Außerdem verbleibt ein mehr oder weniger großer Rest auf dem Mischblock, der nach der Aushärtung verworfen werden muß.

Die Verwendung von eugenolhaltigen Präparaten ist toxikologisch nicht unbedenklich und bei einem Teil der Patienten aufgrund allergischer Reaktionen kontraindiziert. Weiterhin wird der Geruch und der Geschmack von Eugenol häufig als unangenehm empfunden.

Wird zur anschließenden endgültigen Befestigung des Zahnersatzes ein Compositebefestigungszement verwendet, so führen Reste des Zinkoxid-Eugenolzementes in Dentinkanälen sowie auf dem Zahnersatz zu einer Inhibition der Polymerisation und dadurch zu einer ungenügenden Festigkeit. Durch die zunehmende Verwendung von Compositezementen tritt dieses Problem immer stärker in den Vordergrund.

Neben den eugenolhaltigen Präparaten werden zur vorübergehenden Befestigung von Kronen und Brücken auch calciumhydroxidhaltige Zemente verwendet.

Diese zeigen neben den oben angeführten Problemen bei der Verarbeitung von zwei Pasten sowie Wechselwirkung mit Compositbefestigungszementen noch eine ungenügende mechanische Festigkeit und sind daher nur für Kurzzeitprovisorien verwendbar. Zudem ist in jedem Fall ein Arbeitsgang "Anmischen" bei zweikomponentigen Materialien nötig.

GB-A-1 222 568 beschreibt bei Raumtemperatur vernetzende Organopolysiloxan-Elastomere und deren Verwendung unter anderem als Zahnabdruckmasse und Fugendichtstoff in der Bautechnik.

Es wurde nun gefunden, daß bestimmte alkoxyfunktionelle Silikonharze, die durch Feuchtigkeit rasch aushärten, geeignete einkomponentige Formulierungen als provisorische Befestigungsmaterialien erlauben. Die Geschwindigkeit der Aushärtung kann durch Zusatz geeigneter Aktivatoren eingestellt werden. Die mechanische Festigkeit kann je nach vorgesehener Anforderung durch Zusatz von Füllstoffen bzw. Weichmachern in weiten Bereichen verändert werden. Das Material kann durch übliche Pigemente zahnfarben eingefärbt werden. Die Pasten sind geruchs- und geschmackslos. Das ausgehärtete Material verhält sich inert und beeinflußt z.B. nicht die Aushärtung von Compositzementen. Die erfindungsgemäßen Materialien können ohne weitere Bearbeitung beim Zahnarzt eingesetzt werden. Eine Anmischung vor Gebrauch entfällt. Die erfindungsgemäßen alkoxyfunktionellen Silikonharze entsprechen vorzugsweise der Bruttoformel

$$R_xSi(OR')_y O_{\frac{4-x-y}{2}}$$

in der

- R eine Alkyl- oder Phenylgruppe bedeutet,
- R' eine Methyl oder Ethylgruppe bedeutet,
- x einen Wert von 0,75 - 1,75, vorzugsweise 0,95 - 1,5 und
- y einen Wert von 0,3 - 2,0, vorzugsweise 0,5 - 1,7 aufweist.

Die Reste R und R' können jedoch auch verschiedene Reste mit obengenannter Bedeutung darstellen, wie in Beispiel 6 erläutert.

Beispielhaft für erfindungsgemäße Pasten seien hier folgende Zusammensetzungen genannt:

15 - 98 Gew.Tle alkoxyfunktionelles Silikonharz, vorzugsweise 15-40 Gew.-Teile

0 - 85 Gew.Tle Füllstoffe, vorzugsweise 20-70 Gew.-Teile

0 - 50 Gew.Tle Weichmacher, vorzugsweise 2-20 Gew.-Teile

0,1 - 5 Gew.Tle Aktivator, vorzugsweise 0,5-2 Gew.-Teile
0 - 1 Gew.Tle Pigmente
Geeignete Silikonharze sind z.B.:
Methoxy- oder ethoxyfunktionelle Methylsilikonharze, methoxy- oder ethoxyfunktionelle Phenylsilikonharze, sowie deren Mischungen.

Die Silikonharze haben vorzugsweise ein Molekulargewicht von 300 bis 30 000, besonders bevorzugt 800 bis 10 000, ganz besonders bevorzugt 1 000 bis 6 200.

Solche Harze, sowie ihre Herstellung sind beschrieben in W. Noll: Chemie und Technologie der Silikone, Verlag Chemie, 1968.

Bevorzugt enthalten erfindungsgemäße Befestigungsmaterialien 15 - 40 Gew.-% der alkoxylfunktionellen Silikonharze.

Geeignete Füllstoffe sind z.B.:
hochdisperse Kieselsäuren, Metalloxyde wie Zinkoxyd, Magnesiumoxyd oder Aluminiumoxyd; Calciumcarbonat, Calciumfluorid, Glas- oder Quarzpulver sowie Mischungen dieser Füllstoffe. Die Füllstoff können gegebenenfalls hydrophobiert sein. Die Hydrophobierung erfolgt bei $SiO_2$-haltigen Materialien vorzugsweise mit Silanen oder bei basischen Stoffen wie $CaCO_3$ oder ZnO vorzugsweise mit langkettigen Carbonsäuren wie Stearinsäure, Vorzugsweise werden die Füllstoffe in noch getrockneter Form mit Restwassergehalten <0,5 Gew.-% eingesetzt. Als Füllstoffe können auch Kunstoffpulver wie z.B. Poly(meth)-acrylatpulver eingesetzt werden. Die Füllstoffe haben vorzugsweise eine mittlere Korngröße <25 µm, bevorzugt <15 µm, ganz besonders bevorzugt <5 µm.

Geeignete Weichmacher sind z.B., beispielsweise Oligo- oder Polyether und/oder Polyether und/oder Polyetherpolysiloxanverbindungen und/oder silikonharzlösliche Kohlenwasserstoffverbindungen wie z.B :
Silikonöle, Polyetherpolysiloxane, Phenolalkylsulfonsäureester, Polyethylenglycolether oder -ester, Dibenzyltoluol, Paraffine und Isoparaffine (z.B. Isoeikosan) sowie Mischungen dieser Verbindungen.

Geeignete Aktivatoren sind z.B.:
organische Zinn-, Zirkon- oder Titanverbindungen wie Dibutylzinndilaurat, Zinn(II)octoat, Titantetrabutylat, Tetrapropyltitanat, Tetraethyltitanat, entsprechende Alkylzirkonate oder Mischungen dieser Aktivatoren.

Die folgenden Beispiele dienen der Erläuterung der Erfindung:

Beispiel 1

26 Gew.Tle methoxyfunktionelles Methylsilikonharz*
38 Gew.Tle Zinkweiß
12 Gew.Tle hydrophobierte hochdisperse Kieselsäure
21,5 Gew.Tle Isoeicosan
2,5 Gew.Tle Tetrapropyltitanat
werden unter Auschluß von Feuchtigkeit zu einer Paste verarbeitet.

*Bruttoformel:

$$Me\ Si(OMe)_{0,88}\ (O)_{\frac{2,12}{2}}$$

(NMR-spektoskopisch; Me = Methyl)
Viskosität: 40.2 mPas
Dichte : 1.150 g/ml
Die Paste wird in das zu befestigende Objekt (Krone bzw. Brücke) eingebracht und auf den präparierten Zahnstumpf positioniert. Nach 2 min. kann das überschüssige Material entfernt werden. Bei Bedarf können die Objekte ohne Aufwand wieder abgenommen werden und das ausgehärtete spröde Material kann mit der zahnärztlichen Sonde abgesprengt werden.

Beispiel 2:

23 Gew.Tle methoxyfunktionelles Methylsilikonharz (aus Beispiel 1)
30 Gew.Tle Calciumfluorid
25 Gew.Tle hydrophobierte gefüllte Kieselsäure

19 Gew.Tle Isoeicosan
2,5 Gew.Tle Tetrabutyltitanat
0,5 Gew.Tle Pigmente
werden unter Ausschluß von Feuchtigkeit zu einer Paste verarbeitet.

Die Aushärtung des Zements benötigt 3 Minuten (Hautbildung bei 36 ° C und 95 % rel. Feuchte). Das Material hat nach 24 Stunden eine Druckfestigkeit von 15 MPa.

Beispiel 3:

50 Gew.Tle methoxyfunktionelles Methylsilikonharz (aus Beispiel 1)
50 Gew.Tle hydrophobierte Kreide
werden unter Feuchtigkeitsausschluß zu einer Paste verarbeitet.

Die Pasten werden mit unterschiedlichen Mengen Aktivator versetzt und die Aushärtungszeit (Hautbildung bei 36 ° C und 95 % relativer Feuchte) sowie die Druckfestigkeit (nach 24 Stunden bei 23 ° C und 95 % relativer Feuchte) gemessen.

| Gehalt Aktivator (Gew.Tle bezogen auf Silikonharz) | Aushärtungszeit | Druckfestigkeit (MPa) |
|---|---|---|
| 0,5 Dibutylzinndilaurat<br>0,5 Tetrabutyltitanat | 2' | 20 |
| 1 Dibutylzinndilaurat | 7' | 35 |
| 10 Tetrabutyltitanat | 4'30'' | 25 |
| 10 Tetrabutylzirkonat | 5'30'' | 30 |

Beispiel 4:

In eine Lösung von 5 Gew.-% Tetrabutyltitanat in methoxyfunktionellem Silikonharz (aus Beispiel 1) werden unterschiedliche Anteile Füllstoff unter Feuchtigkeitsausschluß eingearbeitet und die Aushärtungszeit (Hautbildung bei 36 ° C und 95 % relativer Feuchtigkeit) sowie die Druckfestigkeit (nach 24 Stunden nach 23 ° C und 95 % relativer Feuchte) gemessen.

| Füllstoff | Aushärtungszeit | Druckfestigkeit (MPa) |
|---|---|---|
| 50 Gew.Tle Lösung +<br>50 Gew.Tle Kreide | 6' | 25 |
| 67 Gew.Tle Lösung +<br>33 Gew.Tle gefällte Kieselsäure | 3'30'' | 30 |
| 90 Gew.Tle Lösung +<br>10 Gew.Tle Acrylesterpolymerisat | 2'30'' | 17 |

Beispiel 5

Eine Lösung von 5 Gew.-% Tetrabutylorthotitanat in methoxyfunktionellem Methylsilikonharz (aus Beispiel 1) wird mit gleichen Teilen hydrophobierter Kreide zu einer Paste verarbeitet. Diese Paste wird mit unterschiedlichen Anteilen Weichmacher versetzt und die Aushärtungszeit (Hautbildung bei 36 ° C und 95 % relativer Feuchte) sowie die Druckfestigkeit (nach 24 Stunden bei 23 ° C und 95 % relativer Feuchte) gemessen.

| Verdünner | Aushärtungszeit | Druckfestigkeit (MPa) |
|---|---|---|
| 7,5 Gew.Tle Triethylenglycoltriacetat | 6' | 11,5 |
| 7 Gew.Tle polyoxalkyliertes Polysiloxan | 14' | 12 |

Beispiel 6:

49,5 Gew.Tle Silikonharz
49,5 Gew.Tle hydrophobierte Kreide
0,5 Gew.Tle Dibutylzinndilaurat
0,5 Gew.Tle Tetrabutyltitanat
werden unter Feuchtigkeitsausschluß zu einer Paste verarbeitet und die Aushärtungszeit (Hautbildung bei 60 % relativer Feuchte und 23 ° C) gemessen.

| Silikonharz | Aushärtungszeit (Min.) | Bruttoformel |
|---|---|---|
| Methoxyfunktionelles Methylsilikonharz | 4' | $(Me)_{1,0}Si(oMe)_{0,88}(O)_{\frac{2,12}{2}}$ |
| Methoxyfunktionelles Methylphenylsilikonharz | 7' | $(Me)_{0,8}(Ph)_{0,7}Si(OMe)_{0,44}(O)_{\frac{2,06}{2}}$ |
| Ethoxyfunktionelles Methylphenylsilikonharz | 7' | $(Me)_{0,4}(Ph)_{0,8}Si(OET)_{0,96}(O)_{\frac{1,84}{2}}$ |
| Methoxyfunktionelles Methylphenylsilikonharz | 45' | $(Me)_{0,7}(Ph)_{0,3}Si(OMe)_{0,84}(O)_{\frac{2,16}{2}}$ |

**Patentansprüche**

**Patentansprüche für folgende Vertragsstaaten : AT, BE, CH, DE, DK, FR, GB, GR, IT, LI, LU, NL, PT, SE**

1. Feuchtigkeitshärtendes provisorisches Befestigungsmaterial, dadurch gekennzeichnet, daß es alkoxyfunktionelle Silikonharze mit der Bruttoformel:

$$R_xSi(OR')_y\,O_{\frac{4-x-y}{2}}$$

wobei
R eine Alkyl- oder Phenylgruppe ist,
R' eine Methyl- oder Ethylgruppe ist,
x einen Wert von 0,75 - 1,75 und
y einen Wert von 0,3 - 2,0 aufweist
sowie einen Aktivator und gegebenenfalls Weichmacher, Füllstoffe und Pigmente enthält.

2. Feuchtigkeitshärtendes Befestigungsmaterial gemäß Anspruch 1, dadurch gekennzeichnet, daß es sich bei dem Aktivator um Titan-, Zirkon- oder Zinnverbindungen handelt.

3. Feuchtigkeitshärtendes Befestigungsmaterial gemäß Anspruch 1, dadurch gekennzeichnet, daß es als Weichmacher Oligo- oder Polyether und/oder Polyetherpolysiloxanverbindungen und/oder siliconharzlösliche Kohlenwasserstoffverbindungen enthält.

4. Feuchtigkeitshärtendes Befestigungsmaterial gemäß Anspruch 1, dadurch gekennzeichnet, daß das Material 15 bis 40 Gew.-% alkoxyfunktionelles Silikonharz enthält.

5. Verwendung eines feuchtigkeitshärtenden Materials gemäß Anspruch 1 enthaltend:
15 - 98 Gew.Tle alkoxyfunktionelles Silikonharz
0 - 85 Gew.Tle Füllstoff
0 - 50 Gew.Tle Weichmacher
0,1 - 5 Gew.Tle Aktivator
0 - 1 Gew.Tle Pigmente
zur provisorischen Befestigung von Zahnersatz an Zähnen,

6. Verwendung des Befestigungsmaterials gemäß Ansprüchen 1 bis 4 bei der Herstellung von Mitteln zur Befestigung von Zahnkronen und -brücken.

**Patentansprüche für folgenden Vertragsstaat : ES**

1. Verwendung eines feuchtigkeitshärtenden provisorischen Befestigungsmaterials, dadurch gekennzeichnet, daß es alkoxyfunktionelle Silikonharze mit der Bruttoformel:

$$R_xSi(OR')_y\,O_{\frac{4-x-y}{2}}$$

wobei
R eine Alkyl- oder Phenylgruppe ist,
R' eine Methyl- oder Ethylgruppe ist,
x einen Wert von 0,75 - 1,75 und
y einen Wert von 0,3 - 2,0 aufweist,
sowie einen Aktivator und gegebenenfalls Weichmacher, Füllstoffe und Pigmente enthält, bei der Herstellung von Mitteln zur Befestigung von Zahnkronen und -brücken.

2. Verwendung gemäß Anspruch 1, dadurch gekennzeichnet, daß es sich bei dem Aktivator um Titan-, Zirkon- oder Zinnverbindungen handelt.

3. Verwendung gemäß Anspruch 1, dadurch gekennzeichnet, daß es als Weichmacher Oligo- oder Polyether und/oder Polyetherpolysiloxanverbindungen und/oder siliconharzlösliche Kohlenwasserstoffverbindungen enthält.

**4.** Verwendung gemäß Anspruch 1, dadurch gekennzeichnet, daß das Material 15 bis 40 Gew.-% alkoxyfunktionelles Silikonharz enthält.

**5.** Verwendung gemäß Anspruch 1, dadurch gekennzeichnet, daß ein feuchtigkeitshärtendes Material, enthaltend:
15 - 98 Gew.-Tle. alkoxyfunktionelles Silikonharz
0 - 85 Gew.-Tle. Füllstoff
0 - 50 Gew.-Tle Weichmacher
0,1 - 5 Gew.-Tle Aktivator
0 - 1 Gew.-Tle Pigmenge
zur provisorischen Befestigung von Zahnersatz an Zähnen eingesetzt wird.

**Claims**
**Claims for the following Contracting States : AT, BE, CH, DE, DK, FR, GB, GR, IT, LI, LU, NL, PT, SE**

**1.** Provisional fixing material which hardens in the presence of moisture, characterised in that it contains silicone resins which contain alkoxy functional groups and have the empirical formula:

$$R_xSi(OR')_yO_{\frac{4-x-y}{2}}$$

where
R is an alkyl or phenyl group,
R' is a methyl or ethyl group,
x has a value of 0.75 - 1.75 and
y has a value of 0.3 - 2.0
as well as an activator and if appropriate plasticisers, fillers and pigments.

**2.** Fixing material which hardens in the presence of moisture according to Claim 1, characterised in that the activator is a titanium, zirconium or tin compound.

**3.** Fixing material which hardens in the presence of moisture according to Claim 1, characterised in that it contains oligo- or polyethers and/or polyetherpolysiloxane compounds and/or hydrocarbon compounds which are soluble in silicone resin as plasticisers.

**4.** Fixing material which hardens in the presence of moisture according to Claim 1, characterised in that the material contains 15 to 40 % by weight of silicone resin containing alkoxy functional groups.

**5.** Use of a material which hardens in the presence of moisture, according to Claim 1, and contains:
15 - 98 parts by weight of silicone resin containing alkoxy functional groups
0 - 85 parts by weight of filler
0 - 50 parts by weight of plasticiser
0.1 - 5 parts by weight of activator
0 - 1 part by weight of pigments
for provisional fixing of a dental prosthesis to teeth.

**6.** Use of the fixing material according to Claims 1 to 4 in the preparation of agents for fixing dental crowns and bridges.

**Claims for the following Contracting State : ES**

1. Use of a provisional fixing material which hardens in the presence of moisture, characterised in that it contains silicone resins which contain alkoxy functional groups and have the empirical formula:

$$R_xSi(OR')_yO_{\frac{4-x-y}{2}}$$

where
R is an alkyl or phenyl group,
R' is a methyl or ethyl group,
x has a value of 0.75 - 1.75 and
y has a value of 0.3 - 2.0
as well as an activator and if appropriate plasticisers, fillers and pigments, in the preparation of agents for fixing dental crowns and bridges.

2. Use according to Claim 1, characterised in that the activator is a titanium, zirconium or tin compound.

3. Use according to Claim 1, characterised in that it contains oligo- or polyethers and/or polyetherpolysiloxane compounds and/or hydrocarbon compounds which are soluble in silicone resin as plasticisers.

4. Use according to Claim 1, characterised in that the material contains 15 to 40 % by weight of silicone resin containing alkoxy functional groups.

5. Use according to Claim 1, characterised in that a material which hardens in the presence of moisture and contains:
15 - 98 parts by weight of silicone resin containing alkoxy functional groups
0 - 85 parts by weight of filler
0 - 50 parts by weight of plasticiser
0.1 - 5 parts by weight of activator
0 - 1 part by weight of pigments
is used for provisional fixing of a dental prosthesis to teeth.

**Revendications**

**Revendications pour les Etats contractants suivants : AT, BE, CH, DE, DK, FR, GB, GR, IT, LI, LU, NL, PT, SE**

1. Matière de fixation provisoire durcissant à l'humidité, caractérisée en ce qu'elle contient des résines de silicone alcoxy fonctionnelles répondant à la formule brute :

$$R_xSi(OR')_yO_{\frac{4-x-y}{2}}$$

dans laquelle
R représente un groupe alkyle ou un groupe phényle,
R' représente un groupe méthyle ou un groupe éthyle,
x présente une valeur de 0,75 - 1,75, et
y présente une valeur de 0,3 - 2,0,
ainsi qu'un activateur et éventuellement des plastifiants, des matières de remplissage et des pigments.

2. Matière de fixation durcissant à l'humidité selon la revendication 1, caractérisée en ce que, quant à l'activateur, il s'agit de composés de titane, de zirconium ou d'étain.

3. Matière de fixation durcissant à l'humidité selon la revendication 1, caractérisée en ce qu'elle contient, à titre de plastifiant, des oligo- ou polyéthers et/ou des composés de polyéther-polysiloxanes et/ou des composés d'hydrocarbures solubles dans de la résine de silicone.

4. Matière de fixation durcissant à l'humidité selon la revendication 1, caractérisée en ce que la matière contient de 15 à 40% en poids de résine de silicone alcoxy fonctionnelle.

5. Utilisation d'une matière durcissant à l'humidité selon la revendication 1, contenant :
15 - 98 parties en poids de résine de silicone alcoxy fonctionnelle
0 - 85 parties en poids de matière de remplissage
0 - 50 parties en poids de plastifiant
0,1 - 5 parties en poids d'activateur,
0 - 1 partie en poids de pigment
pour la fixation provisoire de prothèses dentaires sur des dents.

6. Utilisation de la matière de fixation selon les revendications 1 à 4, dans la préparation d'agents pour la fixation de couronnes et de ponts dentaires.

**Revendications pour l'Etat contractant suivant : ES**

1. Utilisation d'une matière de fixation provisoire durcissant à l'humidité, caractérisée en ce qu'elle contient des résines de silicone alcoxy fonctionnelles répondant à la formule brute :

$$R_xSi(OR')_yO_{\frac{4-x-y}{2}}$$

dans laquelle
R représente un groupe alkyle ou un groupe phényle,
R' représente un groupe méthyle ou un groupe éthyle,
x présente une valeur de 0,5 - 1,75, et
y présente une valeur de 0,3 - 2,0,
ainsi qu'un activateur et éventuellement des plastifiants, des matières de remplissage et des pigments, lors de la préparation d'agents pour la fixation de couronnes et de ponts dentaires.

2. Utilisation selon la revendication 1, caractérisée en ce que, quant à l'activateur, il s'agit de composés de titane, de zirconium ou d'étain.

3. Utilisation selon la revendication 1, caractérisée en ce qu'elle contient, à titre de plastifiant, des oligo- ou polyéthers et/ou des composés de polyéther-polysiloxanes et/ou des composés d'hydrocarbures solubles dans de la résine de silicone.

4. Utilisation selon la revendication 1, caractérisée en ce que la matière contient de 15 à 40% en poids de résine de silicone alcoxy fonctionnelle.

5. Utilisation d'une matière durcissant à l'humidité selon la revendication 1, caractérisée en ce qu'on met en oeuvre une matière durcissant à l'humidité contenant :
15 - 98 parties en poids de résine de silicone alcoxy fonctionnelle
0 - 85 parties en poids de matière de remplissage
0 - 50 parties en poids de plastifiant
0,1 - 5 parties en poids d'activateur,
0 - 1 partie en poids de pigment
pour la fixation provisoire de prothèses dentaires sur des dents.